# EUROPÄISCHE PATENTSCHRIFT

(11) **EP 3 025 581 B1**
(45) Veröffentlichungstag und Bekanntmachung des Hinweises auf die Patenterteilung: **03.10.2018**
(21) Anmeldenummer: 15792125.5
(22) Anmeldetag: 23.03.2015
(51) Int. Cl.: G01N 33/00, G01N 33/22, F41H 11/132

(54) **VERFAHREN ZUR ERKENNUNG VON SPRENGSTOFFEN UND ANDEREN ZIELSUBSTANZEN**
METHOD OF DETECTING EXPLOSIVES AND OTHER TARGET SUBSTANCES
PROCÉDÉ DE DÉTECTION DE SUBSTANCES EXPLOSIVES ET AUTRES SUBSTANCES CIBLES

(30) Priorität: 13.05.2014 RU 2014119145
(43) Veröffentlichungstag der Anmeldung: 01.06.2016
(73) Patentinhaber: Public Joint Stock Company Aeroflot-Russian Airlines, Moscow 119002 (RU)
(72) Erfinder: ZARIPOV, Azat Gumerovich, Moscow 125368 (RU); SULIMOV, Klim Timofeevich, Moscow 125040 (RU); KOGUN, Galina Anatolevna, Gorki-25 Moscow region 141897 (RU); GRIGOREV, Oleg Aleksandrovich, Moscow 122225 (RU); LUKYANOVA, Svetlana Nikolaevna, Moscow 125080 (RU); ALEKSEEVA, Viktoriya Aleksandrovna, Khimki Moscow region 141407 (RU); KOKLIN, Aleksandr Evgenevich, Moscow 123182 (RU)
(74) Vertreter: Jeck, Anton
(86) Internationale Anmeldenummer: PCT/RU2015/000166
(87) Internationale Veröffentlichungsnummer: WO 2015/174888

(56) Entgegenhaltungen:
- EP-A1- 1 942 342
- WO-A1-2014/064693
- RU-A- 2006 134 467
- RU-C1- 2 288 488
- US-A1- 2003 130 568
- US-A1- 2012 111 285
- Ray C Phillips: "TECH1NICAL MEMORANDUM NO. LWL-CR-01B70 TRAINING DOGS FOR EXPLOSIVES DETECTION", , 1 October 1971 (1971-10-01), XP055363854, Retrieved from the Internet: URL:http://www.dtic.mil/dtic/tr/fulltext/u 2/733469.pdf [retrieved on 2017-04-13]

## Beschreibung

Die Erfindung bezieht sich auf ein Verfahren zur Detektion von Spreng- und anderen Zielstoffen mit Hilfe von Spürhunden. Sie kann in Flughäfen, Bahnhöfen und anderen vielfrequentierten öffentlichen Orten angewendet werden.

Der Geruchssinn ist der Hauptsinn des Hundes. Die unübertroffene Entwicklung der Nase des Hundes als Geruchsanalysator ist Voraussetzung für den Einsatz von Diensthunden als Geruchsdetektoren.

Die Erkennung des Geruchs eines Zielstoffs signalisieren die Spürhunde dem Diensthundeführer dadurch, dass sie ihr Verhalten, insbesondere ihre Stellung, ändern (der Hund setzt sich oder legt sich neben die aufgespürte Geruchsquelle). Der Diensthundeführer erkennt visuell diese Verhaltensänderung des Spürhunds, die vermutlich der Aufspürung des jeweiligen Zielstoffgeruchs entspricht.

Durch die Anmeldung US2009/0139459A1 "Canine certification method", IPC A01K29/00, GO1N33/00, Veröffentlichungsdatum 04.06.2009, ist ein Verfahren zur Eignungsprüfung der Spürhunde für die Detektion von Zielstoffen, darunter von Rauschgiften und Sprengstoffen, bekannt. Dabei befinden sich der Hund und der Diensthundeführer in einem abgesonderten Raum, dem die Luft mit einem bestimmten Geruch in einer bekannten Konzentration zugeführt wird. Die Geruchsstärke wird schrittweise erhöht und dabei wird die Reaktion des Hundes auf die bestimmte Geruchskonzentration visuell festgestellt. Das Verfahren hilft dabei, die Spürhunde auszuwählen und die Wahrnehmungsschwelle des Spürhunds zu ermitteln.

Aus dem Artikel von O. Boguslavskaya "Geruch des Bösen" ("Moskovskiy komsomolets", 23.03.2007, http://I 001.ru/arc/mk/issue329/) ist ein Detektionsverfahren für Spreng- und andere Zielstoffe bekannt, dem die visuelle Beobachtung des Verhaltens der Spürhunde zugrunde liegt. Dabei werden Referenz-Geruchsmusterproben in einem Raum untergebracht. Die Musterproben werden dann mit dem ersten Hund umgangen. Die Referenz-Musterprobe mit dem Geruch des Zielstoffs wird nach dem entsprechenden Verhalten des Hunds bestimmt (der Hund setzt sich neben die Musterprobe, die nach dem Zielstoff riecht). Bekanntlich können die Hunde die Detektion der Zielstoffe falsch anzeigen, um vom Diensthundeführer eine Belohnung zu bekommen. Da die Zuverlässigkeit der Detektion des Zielstoffs durch einen Spürhund als nicht hoch genug gilt, verlässt der erste Hund den Raum, die Referenz-Musterproben werden vertauscht, und in den Raum wird der nächste Hund geführt, um das Experiment zu wiederholen. Danach werden die Referenz-Musterproben wiederum vertauscht und die Hunde gewechselt. Zeigen alle Hunde sicher die gleiche Referenz-Musterprobe an, so wird die Detektion des Zielstoffs als positiv beurteilt.

Das beschriebene Verfahren hat den Nachteil, dass mehrere Spürhunde und Diensthundeführer nacheinander eingesetzt werden müssen. Das führt zu einem beachtlichen Sach- und Zeitaufwand.

Aus dem Stand der Technik sind ferner Verfahren zur Ermittlung des psychophysiologischen Zustands des Menschen bekannt. Diese Verfahren beruhen auf dem Zusammenhang zwischen den ablaufenden psychischen Prozessen und der Dynamik der physiologischen Prozesse.

Durch die RU12970U1 "Einrichtung für Aromatherapie", IPC7 A61L9/00, Veröffentlichungsdatum 20.03.2000, ist ein Verfahren zur Überwachung des psychophysiologischen Zustands des Menschen bei Zufuhr des eine bestimmte Konzentration aufweisenden Geruchs bekannt. Das Verfahren wird mit Hilfe eines Elektroenzephalographs, eines Elektrokardiographs, eines Herzfrequenzmessgeräts oder eines Mehrfachschreibers (Polygraphs) durchgeführt. Um die Informationen über die psychophysiologische Zustandsänderung des Menschen beim Einatmen des Geruchs zu bekommen, werden auf der Testperson Sensoren angebracht, zum Beispiel ein Herzfrequenzsensor oder Atemfrequenzsensor. Die Signale der Sensoren werden in einer Prozessoreinheit verarbeitet. Bei dieser Verarbeitung handelt es sich um einen Vergleich der erfassten Daten mit den Daten, die den "normalen" Zustand des Menschen beschreiben.

Aus dem Stand der Technik ist weiterhin ein Verfahren zur Fern-Detektion der Spreng- und anderer Zielstoffe mit Hilfe von Spürhunden bekannt, siehe zum Beispiel das Patent RU2288488C1 "Einrichtung zur Suche nach Menschen unter Verschüttungen und nach Sprengstoffen und Rauschgiftmitteln", IPC GO 1 VI 1/00, Veröffentlichungsdatum 27.11.2006, und Patent RU2426141C1 "Einrichtung zur Suche nach Menschen unter Verschüttungen und nach Sprengstoffen und Rauschgiftmitteln", IPC G01S1/02, Veröffentlichungsdatum 10.08.2011. Dabei wird die Herzfrequenz des Spürhunds ferngemessen. Bei rascher Änderung der Herzfrequenz urteilt man über die Detektion der Spreng- und anderer Zielstoffe durch den Spürhund.

Dieses Verfahren ist durch die ungenügende Zuverlässigkeit der Detektion von Spreng- und anderen Zielstoffen nachteilig, denn der Puls des Spürhunds kann sich sowohl bei der Detektion der Zielstoffe als auch in anderen Situationen ändern, zum Beispiel, wenn der Hund ein lebendes Objekt wahrnimmt.

Durch das Patent RU31718U1 "Einrichtung zur Messung von psychophysiologischen Kenndaten des Menschen", IPC A61B 5/16, 5/02, 3/06, Veröffentlichungsdatum 27.08.2003, ist auch ein System zur Messung von psychophysiologischen Kennwerten des Menschen bekannt. Das System umfasst einen Rechner und an seine Eingänge angeschlossene Herzfrequenz- und Atemsensoren, Elektrokardiogramm-Sensoren (nachfolgend EKG) und Elektroenzephalogramm-Sensoren (nachfolgend EEG).

Das System dient der gleichzeitigen Überwachung des Zustands der Nerven-, Atem- und Herzkreislaufsysteme des Menschen.

Die EP 1 942 342 A1 offenbart einen Spürhund mit einer tragbaren elektronischen Einheit zu versehen. Von der elektronischen Einheit während eines Sucheinsatzes des Spürhundes aufgenommene Daten bezüglich der Körperstellung und bezüglich biometrischer Daten werden drahtlos an eine tragbare Rechnereinheit übertragen und dort von einem Hundeführer ausgewertet.

Die US 2003/0130568 A1 offenbart ein Verfahren zum Beobachten, Sammeln und Auswerten olfaktorischer Charakteristika von Menschen oder Tieren, wie etwa Schnüffeln oder Atmen und die Verwendung dieses Verfahrens in der Ausbildung und Bewertung von Spürhunden.

Die WO 2014/064693 A1 beschreibt einen Apparat zum Trainieren eines Hundes darauf, Detektionshandlungen in einem vollständig abgeschlossenen Abteil vorzunehmen. Hierbei weist das Abteil ein Teilabteil auf, dessen Innenraum durch zumindest drei unterschiedlich gewinkelte Oberflächen definiert ist. Diese Oberflächen sind ausreichend lang, um einem sich im Innenraum befindenden Hund das Gefühl zu geben, zumindest teilweise eingesperrt zu sein. In einer Einstellung des Teilabteils wird geruchsbeladene Luft in den Innenraum geleitet und kann vom Hund wahrgenommen werden. Ein vom Hund bedienbares Element ist operativ mit dem Teilabteil verbunden und kann vom Hund nach Feststellung der Anwesenheit eines Zielgeruchs betätigt werden.

Als nächstkommender Stand der Technik hinsichtlich der vorliegenden Erfindung gilt ein Detektionsverfahren für Spreng- und andere Zielstoffe, gemäß dem das Verhalten des Spürhunds visuell beobachtet wird, s. Patentanmeldung US2012/0111285A1 "Dynamic canine tracking method for hazardous and illicit substances", IPC A01K15/00, Veröffentlichungsdatum 10.05.2012. Das Vorhandensein von Spreng- und anderen Zielstoffen wird durch eine Veränderung des Verhaltens des Spürhundes erkannt.

Der Mangel des Verfahrens ist die niedrige Zuverlässigkeit der Detektion der Zielstoffe.

### Wesen der Erfindung

Die Aufgabe der vorliegenden Erfindung ist die Entwicklung eines Verfahrens zur Detektion der Spreng- und anderer Zielstoffe mit Hilfe von Spürhunden, das durch eine hohe Zuverlässigkeit der Detektion und eine kurze Kontrolldauer gekennzeichnet ist.

Der technische Effekt der beanspruchten Erfindung ist die Steigerung der Zuverlässigkeit bei der Detektion von Spreng- und anderen Zielstoffen mit Hilfe von Spürhunden und die Verminderung des Zeitaufwands für die Kontrolle.

Der genannte technische Effekt wird mit Hilfe des beanspruchten Verfahrensanspruch 1 zur Detektion von Spreng- und anderen Zielstoffen mit Hilfe von Spürhunden erreicht, gemäß dem ein Objekt durch einen Spürhund in Begleitung eines Diensthundeführers kontrolliert wird. Dabei wird die Verhaltensänderung des Spürhunds visuell verfolgt. Liegt eine Verhaltensänderung des Spürhunds vor, so wird über das Vorhandensein von Sprengstoffen und/oder anderen Zielstoffen geurteilt.

Der genannte technische Effekt wird wie folgt erreicht.

Vor der Kontrolle des Objekts wird die ganzheitliche psychophysiologische Reaktion des Spürhunds auf die Geruchserkennung eines Sprengstoffs und/oder eines anderen Zielstoffs mit einer vorgegebenen Konzentration festgestellt. Bei der Verhaltensänderung des Spürhunds während der Kontrolle des jeweiligen Objekts wird die aktuelle ganzheitliche psychophysiologische Reaktion des Spürhunds festgestellt und mit der vorher festgestellten Reaktion auf die Geruchsdetektion verglichen. Aus den Vergleichsergebnissen werden Schlussfolgerungen über die Spürhund-Detektion der Sprengstoffe und/oder anderer Zielstoffe gezogen.

### Kurzbeschreibung der Zeichnungen

Die Erfindung wird nun anhand folgender Figuren näher beschrieben. Es zeigen:
- Fig. 1: eine skizzenhafte Darstellung des Systems zur Erfassung von psychophysiologischen Werten des Spürhunds und
- Fig. 2: eine schematische Anordnung der Systemelemente.

Dabei bezeichnen die jeweiligen Bezugszeichen in den Figuren folgende Komponenten:
- 1: Atemsensor
- 2: EKG-Sensoren
- 3: EEG-Sensoren
- 4: Anschlussadapter für Sensoren
- 5, 6: Mehrfachschreiber-Verstärker (polygraphischer Verstärker)
- 7, 8: Sender für drahtlose Wi-Fi-Netze
- 9: tragbarer Rechner
- 10: Spürhund
- 11: Kappe
- 12: Weste.

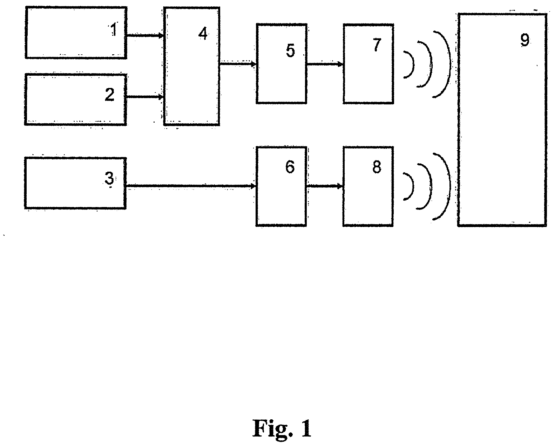

Das Verfahren kann unter Einsatz des Systems zur Erfassung psychophysiologischer Werte des Spürhunds aus Fig. 1 durchgeführt werden.

Das System umfasst einen Atemsensor 1, EKG-Sensoren 2, EEG-Sensoren 3, einen Anschlussadapter 4 für die Sensoren, Mehrfachschreiber-Verstärker 5, 6, Sender 7, 8 für ein drahtloses Wi-Fi-Netz und einen tragbaren Wi-Fi-fähigen Rechner 9.

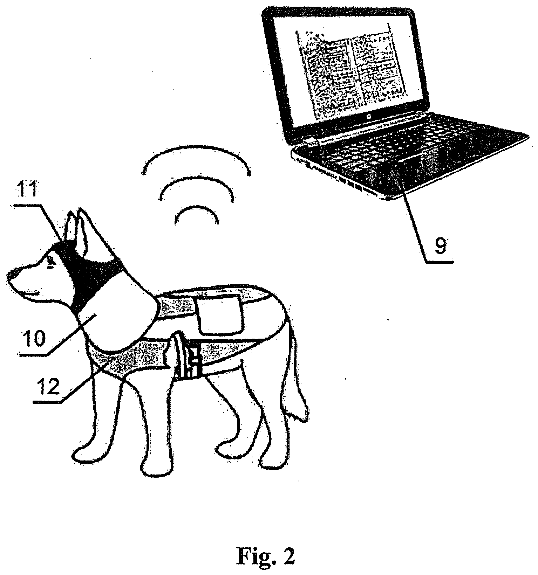

Die aktiven EEG-Sensoren 3 werden auf dem Kopf des Spürhunds 10 angeordnet und auf der Kappe 11 befestigt (Fig. 2).

Die passiven Atemsensoren 1 und EKG-Sensoren 2 werden am Körper des Spürhunds angeordnet und an der Weste 12 befestigt. Auf der Weste 12 werden ebenfalls ein Anschlussadapter 4, Mehrfachschreiber-Verstärker 5, 6 und Sender 7, 8 für das drahtlose Wi-Fi-Netz angebracht.

Um die Erfindung durchzuführen, werden folgende Handlungen in der angegebenen Reihenfolge ausgeführt.

Die Vorbereitung des Spürhunds umfasst folgende Handlungen.

Auf dem Spürhund 10 werden Elemente 1 - 8 des Systems zur Erfassung der psychophysiologischen Werte angeordnet. Neben dem Spürhund 10 in Reichweite des drahtlosen Wi-Fi-Netzes wird ein tragbarer Rechner 9 untergebracht (Laptop, Tablet- Rechner, Smartphone usw.). Dabei soll der Rechner mit drahtlosen Wi-Fi-Netzen kompatibel sein.

Die ganzheitliche psychophysiologische Reaktion des Spürhunds im "normalen" psychophysiologischen Zustand wird mit Hilfe des Systems zur Erfassung von psychophysiologischen Werten erfasst.

Die Geruchsstärke (Konzentration) des Sprengstoffs oder eines anderen Zielstoffs wird mit Hilfe eines Olfaktometers eingestellt (in den Figuren nicht abgebildet).

Der Spürhund wird an der Geruchsmusterprobe mit vorgegebener Konzentration vorbeigeführt. Die Anzeige durch den Spürhund der jeweiligen Geruchsdetektion wird anhand der Verhaltensänderung (der Hund setzt sich) visuell bestimmt.

Die ganzheitliche psychophysiologische Reaktion des Spürhunds auf die Detektion des Geruchs in der Sollkonzentration wird somit ermittelt und im tragbaren Rechner 9 gespeichert.

Während der Kontrolle des jeweiligen Objekts durch den Spürhund werden folgende Handlungen ausgeführt.

Der Spürhund 10 wird mit Elementen 1 - 8 des Systems zur Erfassung der psychophysiologischen Werte versehen. Der Diensthundeführer wird mit einem tragbaren Rechner 9 ausgestattet.

Der Spürhund 10 wird vom Diensthundeführer außerhalb oder innerhalb des jeweiligen untersuchten Objekts begleitet.

Der Diensthundeführer stellt die "Entscheidung" (Anzeige) des Spürhunds über die Geruchsdetektion eines Sprengstoffs und eines anderen Zielstoffs visuell anhand der Verhaltensänderung des Spürhunds fest.

Die aktuelle ganzheitliche psychophysiologische Reaktion des Spürhunds 10 wird mit Hilfe des Systems zur Erfassung der psychophysiologischen Werte ermittelt und im tragbaren Rechner 9 gespeichert.

Die aktuelle ganzheitliche psychophysiologische Reaktion des Spürhunds 10 wird mit der vorher ermittelten und im Speicher des tragbaren Rechners 9 gespeicherten Reaktion verglichen.

Aufgrund der Vergleichsergebnisse der Reaktionen wird über die Spürhund-Detektion der Sprengstoffe und/oder anderer Zielstoffe entschieden.

Die Anwendung der angemeldeten Erfindung in der Praxis hat ihre gewerbliche Anwendbarkeit und die Erreichbarkeit des angegebenen technischen Effekts nachgewiesen.

Die Zuverlässigkeit der Detektion der Spreng- und anderer Zielstoffe mit Hilfe von Spürhunden unter Anwendung der beanspruchten Erfindung wird dadurch erhöht, dass die eigentliche Detektion mit Hilfe der ganzheitlichen psychophysiologischen Reaktion des Körpers des Spürhunds nachgewiesen wird.

Die verkürzte Kontrolldauer wird dadurch erreicht, dass die tatsächliche Detektion der Spreng- und anderer Zielstoffe unabhängig vom Verhalten des Spürhunds durch das technische System zur Erfassung der psychophysiologischen Werte nachgewiesen wird.

## Patentansprüche

1. Detektionsverfahren für Sprengstoffe und/oder andere Zielstoffe mit Hilfe eines Spürhunds (10), wonach ein jeweiliges Objekt mit Hilfe eines Spürhunds (10) in Begleitung eines Diensthundeführers kontrolliert wird, indem die Verhaltensänderung des Spürhunds (10) visuell beobachtet wird, und wonach das Vorhandensein der Sprengstoffe und/oder anderer Zielstoffe anhand der Verhaltensänderung des Spürhunds (10) festgestellt wird,
**dadurch gekennzeichnet,**
**dass** vor der Kontrolle des jeweiligen Objekts eine ganzheitliche psychophysiologische Reaktion des Spürhundes (10) auf die Detektion des Geruchs eines Sprengstoffs und/oder eines anderen Zielstoffs in einer vorgegebenen Konzentration mit einem an dem Spürhund (10) angebrachten System zur Erfassung psychophysiologischer Werte ermittelt wird, wobei die Geruchsstärke (Konzentration) des Sprengstoffs oder eines anderen Zielstoffs mit Hilfe eines Olfaktometers eingestellt wird, dass bei der Verhaltensänderung des Spürhunds (10) während der Kontrolle des jeweiligen Objekts die aktuelle ganzheitliche psychophysiologische Reaktion des Spürhunds (10) mit dem an dem Spürhund (10) angebrachten System zur Erfassung psychophysiologischer Werte festgestellt und mit der vorher ermittelten Reaktion auf die Geruchsdetektion verglichen wird,
**dass** das an dem Spürhund (10) angebrachte System Elektrokardiogramm-Sensoren (2), Elektroenzephalogramm-Sensoren (3) und einen Atemsensor (1) umfasst,
**dass** die ganzheitliche psychophysiologische Reaktion des Spürhundes (10) auf die Detektion des Geruchs des Sprengstoffs und/oder des anderen Zielstoffs in der vorgegebenen Konzentration in einem tragbaren Rechner (9) gespeichert wird,
**dass** die aktuelle ganzheitliche psychophysiologische Reaktion des Spürhunds (10) bei der Kontrolle des Objekts in dem tragbaren Rechner (9) gespeichert wird,
**dass** die im Rechner (9) gespeicherte aktuelle ganzheitliche psychophysiologische Reaktion des Spürhunds (10) mit der vorher ermittelten, im Rechner (9) gespeicherten ganzheitlichen psychophysiologischen Reaktion des Spürhunds (10) bei der Detektion des Geruchs des Sprengstoffs und/oder des anderen Zielstoffs in der vorgegebenen Konzentration verglichen wird, um das Vergleichsergebnis zu erhalten und
**dass** anhand der Vergleichsergebnisse über die Detektion durch den Spürhund (10) der Sprengstoffe und/oder anderer Zielstoffe geurteilt wird.

## Claims

1. A detection method for explosives and/or other target substances with the aid of a tracker dog (10), in which a respective object is checked with the aid of a tracker dog (10) accompanied by a canine handler, by visually observing the behavioural change of the tracker dog (10), and in which the presence of the explosives and/or other target substances is determined by the behavioural change of the tracker dog (10),
**characterised in that**
prior to checking the respective object, an integrated psychophysiological reaction of the tracker dog (10) to the detection of the odour of an explosive and/or another target substance in a predetermined concentration is determined using a system, attached to the tracker dog (10), for detecting psychophysiological values, the odour strength (concentration) of the explosive or another target substance being adjusted with the aid of an olfactometer, **in that** if the behaviour of the tracker dog (10) changes when checking the respective object, the current integrated psychophysiological response of the tracker dog (10) is detected by the system, attached to the tracker dog (10), for detecting psychophysiological values and compared with the previously detected response to the odour detection,
**in that** the system attached to the tracker dog (10) comprises electrocardiogram sensors (2), electroencephalogram sensors (3) and a respiratory sensor (1), **in that** the integrated psychophysiological response of the tracker dog (10) to the detection of the odour of the explosive and/or the other target substance in the predetermined concentration is stored in a portable computer (9), **in that** the current integrated psychophysiological response of the tracker dog (10) when checking the object is stored in the portable computer (9), **in that** the current integrated psychophysiological response of the tracker dog (10), which response is stored in the computer (9), is compared with the previously determined integrated psychophysiological response, stored in the computer (9), of the tracker dog (10) upon detection of the odour of the explosive and/or the other target substance in the predetermined concentration to obtain the comparison result, and
**in that** a judgement is made about the detection by the tracker dog (10) of the explosives and/or other target substances on the basis of the comparison results.

## Revendications

1. Procédé de détection d'explosifs et/ou d'autres substances cibles à l'aide d'un chien détecteur (10), selon laquelle un objet particulier est contrôlé à l'aide d'un chien détecteur (10) accompagné d'un maître-chien, le changement du comportement du chien détecteur (10) étant observé de manière visuelle, et selon lequel la présence d'explosifs et/ou autres substances cibles est constatée à partir du changement de comportement du chien détecteur (10),
**caractérisé en ce qu'**
avant le contrôle de l'objet concerné, une réaction psychophysiologique intégrale du chien détecteur (10) à la détection de l'odeur d'un explosif et/ou d'une autre substance cible à une concentration donnée est déterminée avec un système posé sur le chien détecteur (10) afin de saisir des valeurs psychophysiologiques, l'intensité de l'odeur (la concentration) de l'explosif ou d'une autre substance cible étant ajustée à l'aide d'un olfactomètre, **en ce que**, lors du changement de comportement du chien détecteur (10) pendant le contrôle de l'objet concerné, la réaction actuelle psychophysiologique intégrale du chien détecteur (10) est constatée avec le système posé sur le chien détecteur pour saisir des valeurs psychophysiologiques et est comparée avec la réaction à la détection d'odeur précédemment déterminée,
**en ce que** le système posé sur le chien détecteur (10) comprend des capteurs électrocardiogrammes (2), des capteurs électroencéphalogrammes (3) et un capteur de fréquence respiratoire (1), **en ce que** la réaction psychophysiologique intégrale du chien détecteur (10) à la détection de l'odeur de l'explosif et/ou d'un autre substance cible à la concentration donnée est enregistrée sur un ordinateur portable (9), **en ce que** la réaction actuelle psychophysiologique intégrale du chien détecteur (10) lors du contrôle de l'objet est enregistrée sur l'ordinateur portable (9), **en ce que** la réaction actuelle psychophysiologique intégrale du chien détecteur (10) enregistrée sur l'ordinateur (9) est comparée à la réaction psychophysiologique intégrale du chien détecteur (10) précédemment enregistrée sur l'ordinateur (9) lors de la détection de l'odeur de l'explosif et/ou de l'autre substance cible à la concentration donnée, afin d'obtenir le résultat de la comparaison et
**en ce que**, à partir des résultats de la comparaison, la détection par le chien détecteur (10) des explosifs et/ou d'autres substance de visée est jugée.
